(19) Europäisches Patentamt
European Patent Office
Office européen des brevets

(11) Veröffentlichungsnummer: **0 019 158**
**B1**

(12) **EUROPÄISCHE PATENTSCHRIFT**

(45) Veröffentlichungstag der Patentschrift: **15.09.82**

(51) Int. Cl.³: **C 07 C 85/02**, C 07 C 87/62

(21) Anmeldenummer: **80102378.9**

(22) Anmeldetag: **02.05.80**

(54) **Verfahren zur Herstellung von m-substituierten N-Methylanilinen.**

(30) Priorität: **16.05.79 DE 2919741**

(43) Veröffentlichungstag der Anmeldung: **26.11.80 Patentblatt 80/24**

(45) Bekanntmachung des Hinweises auf die Patenterteilung: **15.09.82 Patentblatt 82/37**

(84) Benannte Vertragsstaaten: **BE CH DE FR GB LI**

(56) Entgegenhaltungen:
**EP-A-0 006 590**
**DD-A-117 446**
**DE-A-2 651 578**
**DE-B-2 061 709**
**US-A-4 029 707**

(73) Patentinhaber: **BASF Aktiengesellschaft, Carl-Bosch-Strasse 38, D-6700 Ludwigshafen (DE)**

(72) Erfinder: **Schirmer, Ulrich, Dr., Berghalde 79, D-6900 Heidelberg 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 019 158 B1

BUNDESDRUCKEREI BERLIN

Verfahren zur Herstellung von m-substituierten N-Methylanilinen

Die Erfindung betrifft ein neues Verfahren zur Herstellung von m-substituierten Methylanilinen durch einbadige Umsetzung von Anilinen mit Ameisensäure in Gegenwart organischer Lösungsmittel und dann mit quaternären Salzen, Alkylierungsmitteln und Alkalihydroxid bei zwei unterschiedlichen Temperaturen.

Es ist aus DD-PS 117 446 bekannt, daß man Acetanilid und Dimethylsulfat in Gegenwart von Tetra-n-butylammoniumchlorid und Benzol mit Natronlauge bei Raumtemperatur umsetzt und in einer Ausbeute von 84 Prozent N-Acetyl-n-methylanilin erhält. Es wird angegeben, daß Katalysatoren eine leichte Alkylierung zur entsprechenden N-Acetyl-N-Alkylverbindung ermöglichen. Setzt man als Ausgangsstoffe ein Anilin mit Ameisensäure um, so muß, wie Beispiel 5 lehrt, das Gemisch zuerst in Gegenwart von Benzol erhitzt, dann Natronlauge und Dimethylsulfat zugesetzt und das Gemisch unterhalb oder bei 25° C gehalten werden. Die wäßrige Phase des Gemischs wird nun mit Benzol ausgeschüttelt und die beiden organischen Lösungen werden dann vereinigt. Anschließend muß das Gemisch unter Zusatz von konzentrierter Salzsäure zum Sieden erhitzt und 90 Minuten am Rückfluß erhitzt werden, um zum N-Methylanilin hydrolysiert zu werden. Wie Beispiel 1 lehrt, ist für die Hydrolyse von N-Methyl-N-Acyl-verbindungen ein Kochen des Endstoffs mit Mineralsäure notwendig. Verwendet man Äthylierungs- und Propylierungsmittel (Beispiele 2 und 3), so erhält man ebenfalls N-Acyl-N-alkylverbindungen. Wie Beispiel 4 zeigt, wird erst nach einer Reaktionszeit von über 16 Stunden bei Kochtemperatur N-Butylanilin in unbefriedigender Ausbeute erhalten.

Eine entsprechende Lehre gibt auch Synthesis, 1976, Seiten 113 und 114. Es wird darauf hingewiesen, daß insbesondere die Abspaltung des Formylrestes nur sehr langsam verläuft; auch nach 22stündiger Reaktion bei 80° C konnten aus dem sauren Auszug der Reaktion nicht mehr als 10 Prozent basische Nebenprodukte isoliert werden. Im Falle von N-Formylanilinen wird gezeigt, daß mit Alkylierungsmitteln, z. B. Dimethylsulfat, sowohl in Gegenwart wie in Abwesenheit eines Katalysators leicht N-Methyl-N-Formylaniline ohne Abspaltung der Acylgruppe erhalten werden.

Es wurde nun gefunden, daß man m-substituierte N-Methylaniline der Formel

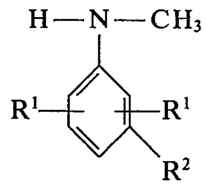

I

worin die einzelnen Reste $R^1$ und $R^2$ gleich oder verschieden sein können und jeweils einen aliphatischen Rest, eine Nitrogruppe, Alkoxygruppe, ein Halogenatom bedeuten, $R^1$ auch ein Wasserstoffatom bezeichnet, durch Alkylierung von Anilinen in Gegenwart von quaternären Ammonium- oder Phosphoniumsalzen, vorteilhaft erhält, wenn man m-substituierte Aniline der Formel

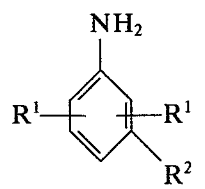

II

worin $R^1$ und $R^2$ die vorgenannte Bedeutung besitzen,

a) mit Ameisensäure in Gegenwart unter den Reaktionsbedingungen inerter, organischer Lösungsmittel bei einer Temperatur unterhalb 140° C umsetzt, anschließend
b) das Gemisch in Gegenwart eines quaternären Salzes der Formel

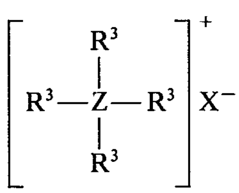

III

worin Z ein Stickstoffatom oder ein Phosphoratom bedeutet, X ein Säureanion bezeichnet und die einzelnen Reste $R^3$ gleich oder verschieden sein können und jeweils für einen aliphatischen, cycloaliphatischen, araliphatischen oder aromatischen Rest stehen, mit einem Methylierungsmittel und Alkalihydroxid bei einer Temperatur von 0 bis 50° C umsetzt und dann

c) das Gemisch bei einer Temperatur oberhalb von 50° C und einem pH oberhalb 13 umsetzt.

Die Umsetzung wird im Falle der Verwendung von m-Nitranilin, Dimethylsulfat und Natronlauge durch die folgenden Formeln wiedergegeben:

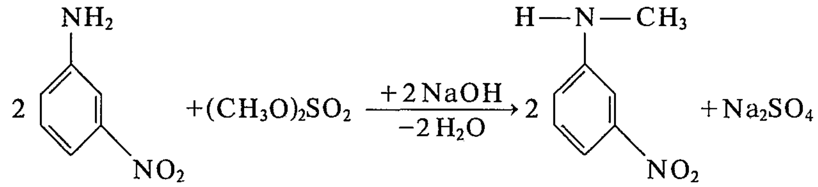


Im Vergleich zu den bekannten Verfahren liefert das Verfahren nach der Erfindung auf einfacherem und wirtschaftlicherem Wege m-substituierte N-Methylaniline in besserer Ausbeute, Reinheit und im Zusammenhang mit der Aufarbeitung bessere Raum-Zeit-Ausbeute. Ein langfristiges Kochen der Reaktionsgemische mit Mineralsäure wird vermieden. Da anstelle von Mineralsäuren Laugen verwendet werden, werden die spätere Neutralisation der Säure und die so anfallenden Salzmengen vermieden und Abwasserprobleme verringert; das erfindungsgemäße Verfahren ist umweltfreundlicher. Alle diese vorteilhaften Ergebnisse sind überraschend. Es war im Hinblick auf den Stand der Technik nicht zu erwarten, daß gerade im Falle der Verwendung von Ameisensäure die erfindungsgemäße Methylierung leicht und mit hohen Ausbeuten durchgeführt werden kann. Es war weiterhin überraschend, daß die Umsetzung auch ohne Verwendung N-acylierter Aniline als Ausgangsstoffe glatt verläuft bzw. eine erhebliche Bildung von N-Acylverbindungen und N-Methyl-N-acylverbindungen als Nebenstoffen auch ohne Kochen des Reaktionsgemisches mit Mineralsäure vermieden wird. Zumindest mußte man die Bildung heterogener Gemische von unumgesetztem, acylierten, methylierten und gleichzeitig acyliertem und methyliertem Anilin erwarten.

Die Ausgangsstoffe II werden im Schritt a) mit Ameisensäure in stöchiometrischer Menge oder im Überschuß, zweckmäßig in einem Verhältnis von 1 bis 5, insbesondere von 1 bis 2 Mol Ameisensäure je Mol Ausgangsstoff II umgesetzt. Bevorzugte Ausgangsstoffe II und dementsprechend bevorzugte Endstoffe I sind solche, in deren Formeln die einzelnen Reste $R^1$ und $R^2$ gleich oder verschieden sein können und jeweils einen Alkylrest oder eine Alkoxygruppe mit jeweils 1 bis 6 Kohlenstoffatomen, eine Trifluormethylgruppe, eine Nitrogruppe, ein Fluoratom, ein Bromatom oder ein Chloratom bedeuten, $R^1$ auch ein Wasserstoffatom bezeichnet. Die vorgenannten Reste können noch durch unter den Reaktionsbedingungen inerte Gruppen, z. B. Alkylgruppen oder Alkoxygruppen mit jeweils 1 bis 4 Kohlenstoffatomen, substituiert sein.

Es können z. B. folgende m-substituierte Aniline als Ausgangsstoffe II verwendet werden: m-Nitro-, m-Chlor-, m-Brom-, m-Fluor-, m-Methyl-, m-Äthyl-, m-Propyl-, m-Isopropyl-, m-Butyl-, m-Isobutyl-, m-sek.-Butyl-, m-tert.-Butyl-, m-Methoxy-, m-Äthoxy-, m-Propoxy-, m-Isopropoxy-, m-Butoxy-, m-Isobutoxy-, m-sek.-Butoxy-, m-tert.-Butoxyanilin; entsprechende, in 2-, 4-, 5-, 6-Stellung einfach oder in 2,4-, 2,5-, 2,6-, 4,5-, 4,6-, 5,6-Stellung zweifach gleich oder unterschiedlich durch die Nitro-, Chlor-, Brom-, Fluor-, Methyl-, Äthyl-, Propyl-, Isopropyl-, Butyl-, Isobutyl-, sek.-Butyl-, tert.-Butyl-, Methoxy-, Äthoxy-, Propoxy-, Isopropoxy-, Butoxy-, Isobutoxy-, sek.-Butoxy-, tert.-Butoxy-gruppe substituierte Aniline mit vorgenannten m-Substituenten; bevorzugt sind 3-Nitro-, 3-Trifluormethyl-, 3-Chlor-4-fluor-, 3-Methoxy-, 3-Methyl-4-fluor-, 3-Chlor-, 3,4-Difluor-, 3-Methyl-5-äthyl-anilin.

Die Umsetzung wird im Schritt a) im allgemeinen bei einer Temperatur von 50 bis 140, vorzugsweise von 80 bis 120°C, drucklos oder unter Druck, diskontinuierlich oder vorzugsweise kontinuierlich durchgeführt. Man verwendet unter den Reaktionsbedingungen inerte, organische Lösungsmittel, z. B. kommen in Frage: aromatische Kohlenwasserstoffe, z. B. Toluol, Äthylbenzol, o-, m-, p-Xylol, o-, m-, p-Diäthylbenzol, Isopropylbenzol, Mesitylen, Methylnaphthalin; Halogenkohlenwasserstoffe, insbesondere Chlorkohlenwasserstoffe, z. B. Tetrachloräthylen, 1,1,2,2- oder 1,1,1,2-Tetrachloräthan, Chloroform, Chlornaphthalin, Dichlornaphthalin, Tetrachlorkohlenstoff, 1,1,1- oder 1,1,2-Trichloräthan, Trichloräthylen, Pentachloräthan, 1,1-Dichloräthan, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol; Nitrokohlenwasserstoffe wie Nitrobenzol, o-, m-, p-Chlornitrobenzol, o-Nitrotoluol; aliphatische oder cycloaliphatische Kohlenwasserstoffe, z. B. Heptan, Pinan, Nonan, Benzinfraktionen innerhalb eines Siedepunktintervalls von 70 bis 190°C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan; N-Methylpyrrolidon, Dimethylformamid; und entsprechende Gemische. Zweckmäßig verwendet man das Lösungsmittel in einer Menge von 200 bis 10 000 Gewichtsprozent, vorzugsweise von 200 bis 1000 Gewichtsprozent, bezogen auf Ausgangsstoff II.

Schritt b) folgt anschließend an Schritt a) und Schritt c) entsprechend an b) ohne Isolierung eines Zwischenproduktes des Ausgangsstoffs aus dem Reaktionsgemisch. Bevorzugt verwendet man im Schritt b) als Alkalihydroxid Kaliumhydroxid und insbesondere Natriumhydroxid. Die Alkalihydroxide werden zweckmäßig in Gestalt von Alkalilaugen verwendet. Man kann im Schritt b) und/oder im

Schritt c) zusätzlich Wasser und/oder unter den Reaktionsbedingungen inerte, organische Lösungsmittel, in der Regel die schon bei Schritt a) verwendeten Lösungsmittel, am Anfang oder während des Schrittes b) bzw. am Anfang oder während des Schrittes c) zusetzen oder die gesamte Lösungsmittelmenge nur auf die Schritte a) und b) verteilen oder die Gesamtmenge am Anfang von Schritt a) zugeben. Zweckmäßig wird man Wasser im Schritt b) und gegebenenfalls im Schritt c) zusammen mit dem Alkalihydroxid in Gestalt von Alkalilauge zuführen. In der Regel wird der Schritt b) des Verfahrens bei einer Temperatur zwischen 0 und 50° C, vorzugsweise 20 und 40° C, der Schritt c) zwischen 50 und 140° C, vorzugsweise zwischen 80 und 120° C, durchgeführt. Man kann jeden Schritt b) oder c) drucklos oder unter Druck, kontinuierlich oder diskontinuierlich durchführen.

Im Schritt b) wird das im Schritt a) gebildete Reaktionsgemisch in Gegenwart quaternärer Ammoniumsalze oder Phosphoniumsalze, zweckmäßig in einer Menge von 0,001 bis 0,1, vorzugsweise 0,005 bis 0,02 Mol quartärem Salz je Mol Ausgangsstoff II umgesetzt. Zweckmäßig sind quartäre Salze der Formel III, worin die einzelnen Reste $R^3$ gleich oder verschieden sein können und jeweils einen Alkylrest mit 1 bis 18, insbesondere 1 bis 7 Kohlenstoffatomen, einen Hydroxyalkylrest, insbesondere einen Hydroxyalkylrest mit 2 bis 18, insbesondere 2 bis 7 Kohlenstoffatomen, einen Cycloalkylrest mit 5 bis 7 Kohlenstoffatomen, einen Aralkylrest mit 7 bis 12 Kohlenstoffatomen oder einen Phenylrest bedeuten, Z für ein Stickstoffatom oder ein Phosphoratom steht, X ein Säureanion bezeichnet. Es können zweckmäßig Salze anorganischer Säuren oder von Sulfonsäuren verwendet werden. Anstelle einbasischer Säuren können auch äquivalente Mengen mehrbasischer Säuren zur Anwendung gelangen. Von mehrbasischen, insbesondere zweibasischen Säuren, kommen mono-, di- oder polybasische Salze, z. B. das Sulfat, Bisulfat, Phosphat, Hydrogenphosphat, Dihydrogenphosphat, in Frage. Beispielsweise sind die quaternären Salze folgender Säuren geeignet: Salzsäure, Bromwasserstoffsäure, Jodwasserstoffsäure, Fluorwasserstoffsäure, Perchlorsäure, Schwefelsäure, Phosphorsäure, Salpetersäure, Sulfonsäuren wie Methylsulfonsäure, Monomethylsulfat, Benzol- und p-Toluol-sulfonsäure; Tetrafluorborsäure, Trifluormethansulfonsäure, $C_8F_{17}SO_3H$, Hexafluorphosphorsäure; bevorzugt sind die Anionen von Salzsäure, Jodwasserstoff, Schwefelsäure, Bromwasserstoff, Perchlorsäure, Fluorwasserstoff.

Es kommen z. B. als quartäre Salze in Frage: Tetramethyl-, Tetraäthyl-, Tetra-n-propyl-, Tetraisopropyl-, Tetra-n-butyl-, Tetraisobutyl-, Tetra-sek.-butyl-, Tetra-tert.-butyl-, Tetrapentyl-, Tetrahexyl-, Tetra-n-heptyl-, Tetraoctyl-, Tetranonyl-, Tetradecyl-, Tetraundecyl-, Tetradodecyl-ammoniumchlorid; (Tetra-β-hydroxyäthyl)-ammoniumchlorid, (Tetra-γ-hydroxypropyl)-ammoniumchlorid, (Tetra-β-hydroxypropyl)-ammoniumchlorid; durch quartäre Substituierung mit vorgenannten Substituenten und/oder mit der Phenyl-, Benzyl-, Cyclohexyl-, Toluyl-, Methylcyclohexyl-, Phenyläthyl-, Phenylpropyl-, Phenylbutyl-gruppe am Stickstoffatom sich entsprechend aus Anilin, Benzylamin, o,m,p-Toluidin, Triphenyl-, Tribenzyl-, Tricyclohexyl-, Tri(methylcyclohexyl)-, Tri(phenyläthyl)-, Tri(phenylpropyl)-, Tri(phenylbutyl)-amin, in 2-, 3-, 4-Stellung einfach oder in 2,4-, 2,3-, 2,6-, 2,5-, 3,4-, 2,5-Stellung zweifach durch die Methylgruppe an jedem Phenylring substituiertem Triphenylamin ergebende Ammoniumchloride, die auch Ammoniumchloride mit 4 vorgenannten, aber völlig oder teilweise unterschiedlichen Resten sein können, z. B. die sich durch Substituierung mit dem Methylrest aus N,N-Dimethylanilin, N-Methyl-N,N-diäthylamin, N,N-Dicyclohexyl-N-methyl-amin, N-Methyl-N-äthyl-N-n-propylamin, ergebenden quartären Ammoniumchloride; Dimethylbenzyldodecyl-, Cetyltrimethyl-, Methyltriäthyl-, Dimethyldiphenyl-, Trimethyl(o-tert.-butylphenyl)-, β-Äthoxyäthyl-dimethyl-lauryl-, Triäthyl-dodecyl-, Trimethyl-tridecyl-, Trimethyl-diphenyl-methyl-, Trimethyl-n-dodecyl-, Trimethyl-β-hydroxyäthyl-, n-Propyl-trimethyl-, Isoamyl-trimethyl-, Benzyl-dimethyl-n-octyl-, Benzyltrimethyl-, Benzyltriäthyl-, Phenyltrimethyl-, Dimethyldodecyl-phenyl-, Trimethyl-(phenyl-(1)-äthyl)-, Trimethyl-(phenyl-(2)-äthyl)-ammoniumchlorid; analoge Phosphoniumchloride; analoge Jodide, Hydrogensulfate, Bromide, Perchlorate, Fluoride, Nitrate, p-Toluolsulfonate.

Bevorzugte Alkylierungsmittel sind solche der allgemeinen Formel

$$CH_3—Y \qquad IV$$

in der Y ein Chlor- oder Bromatom, den Rest

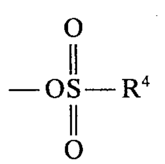

in dem $R^4$ ein Fluoratom, einen aromatischen Rest, vorteilhaft einen gegebenenfalls mit Alkyl- oder Alkoxygruppen mit 1 bis 4 Kohlenstoffatomen oder Chloratomen substituierten Phenyl- oder Tolylrest, oder den Rest

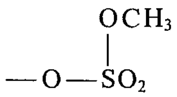

bezeichnet. Zweckmäßig verwendet man Ausgangsstoffe IV in einer Menge von 1 bis 3, vorzugsweise von 1 bis 1,5 Mol je Mol Ausgangsstoff II. Es können z. B. folgende Ausgangsstoffe IV verwendet werden: Methylchlorid, -bromid oder -jodid; Dimethylsulfat; Methylester der Benzol-, Toluol-, 2,5-Dimethylbenzol-, 4-Chlorbenzol-, 4-Methoxy-benzolsulfonsäure, Fluorsulfonsäure.

Die Reaktion kann wie folgt durchgeführt werden: Ein Gemisch von Ausgangsstoff II, Ameisensäure und organischem Lösungsmittel wird unter guter Durchmischung während 0,5 bis 8 Stunden bei der Reaktionstemperatur gehalten. Dem Gemisch werden dann, ohne Abtrennung eines Zwischenproduktes, gegebenenfalls unter Abkühlen auf den angegebenen Temperaturbereich des zweiten Schrittes, Alkalilauge, Salz III, Alkylierungsmittel IV und gegebenenfalls weitere Mengen an Wasser und/oder organischem Lösungsmittel zugegeben. Das Gemisch wird während 0,5 bis 8 Stunden bei dem vorgenannten pH und der Reaktionstemperatur von Schritt b) gehalten, dann auf die Reaktionstemperatur und, gegebenenfalls unter Zuführung von weiterer Alkalilauge und/oder Wasser und/oder organischem Lösungsmittel, auf das vorgenannte pH des Schrittes c) gebracht. Nun wird das Gemisch von 0,5 bis 8 Stunden bei der Reaktionstemperatur von c) gehalten. Dann kann der Endstoff aus dem Gemisch in üblicher Weise, z. B. durc. Filtration, abgetrennt werden.

Die nach dem Verfahren der Erfindung herstellbaren m-substituierten N-Methylaniline I sind wertvolle Ausgangsstoffe für die Herstellung von Farbstoffen, Pflanzenschutzmitteln und Pharmazeutika. Bezüglich der Verwendung wird auf vorgenannte Literatur und die DE-OS 2 703 838 und DE-OS 2 725 146 hingewiesen.

Die in den folgenden Beispielen genannten Teile bedeuten Gewichtsteile.

## Beispiel 1

a) 571 Teile m-Nitranilin, 1500 Teile Toluol, 230 Teile Ameisensäure werden während 4 Stunden am Wasserabscheider zum Sieden (110°C) erhitzt. Nach Auskreisen von 22 Teilen Wasser und 40 Teilen Ameisensäure wird das Gemisch auf 30°C abgekühlt, mit 10 Teilen Triäthylbenzylammoniumchlorid, 630 Teilen Dimethylsulfat und 250 Teilen Toluol versetzt. Unter Kühlung und intensivem Rühren bei 25°C werden 230 Teile Natriumhydroxid in 320 Teilen Wasser innerhalb einer Stunde zugegeben. Nach Nachrühren bei 25°C während einer Stunde wird das Gemisch während 4 Stunden zum Sieden erhitzt (105°C) und das abdestillierende Toluol durch 1500 Teile 20gewichtsprozentige Natronlauge ersetzt. Sobald die gesamte Menge Toluol abdestilliert ist, läßt man das Gemisch unter Rühren abkühlen, saugt ab und wäscht das Festgut mit Wasser nach und trocknet. Man erhält 617 Teile (98% der Theorie) N-Methyl-3-nitranilin vom Fp 66 bis 67°C.

b) Vergleich: Die Umsetzung wird analog Beispiel 1a), aber ohne Zusatz von Triäthylbenzylammoniumchlorid durchgeführt. Man erhält 603 Teile eines Gemisches aus 65 Gewichtsprozent N-Methyl-3-nitranilin (62% der Theorie) und 35 Gewichtsprozent 3-Nitranilin.

c) Vergleich: 786 Teile N-Acetyl-3-nitranilin werden mit 10 Teilen Triäthylbenzylammoniumchlorid, 630 Teilen Dimethylsulfat und 250 Teile Toluol versetzt, und unter Kühlung und intensivem Rühren bei 25°C 230 Teile Natriumhydroxid in 320 Teilen Wasser innerhalb einer Stunde zugegeben. Nach kurzem Nachrühren wird das Gemisch zum Sieden erhitzt und das abdestillierende Toluol durch 20gewichtsprozentige Natronlauge ersetzt. Sobald die gesamte Menge Toluol abdestilliert ist, läßt man unter Rühren abkühlen, saugt das Gemisch ab, wäscht das Festgut mit Wasser nach und trocknet. Man erhält 621 Teile eines Gemisches aus 15 Gewichtsprozent Nitranilin, 80 Gewichtsprozent N-Methyl-3-nitranilin (74,8% der Theorie) und 5 Prozent acylierte Verbindungen.

## Beispiele 2 bis 6

Substituiertes Anilin II (A), Toluol (B), Ameisensäure (C) werden während 4 Stunden am Wasserabscheider zum Sieden (110°C) erhitzt. Nach Auskreisen des Reaktionswassers und der überschüssigen Ameisensäure wird auf 30°C abgekühlt, mit Triäthylbenzylammoniumchlorid (D) und Dimethylsulfat (E) versetzt. Unter Kühlung und intensivem Rühren bei 25°C werden Natriumhydroxid (F) in der 1,5fachen Gewichtsmenge Wasser innerhalb einer Stunde zugegeben. Nach einstündigem Nachrühren bei 25°C wird dem Gemisch 20prozentige Natronlauge (G) zugesetzt und das Gemisch 4 Stunden unter kräftigem Rühren zum Sieden (105°C) erhitzt. Nach dem Abkühlen wird mit Wasser verdünnt, die organische Phase abgetrennt, getrocknet und destilliert. Man erhält so die entsprechenden N-Methylaniline I (H).

In folgender Tabelle sind die Ergebnisse und die verwendeten Mengen der Reaktionskomponenten angegeben.

Tabelle

| Beispiel | $R^1$ | $R^2$ | Ausgangsstoff II (A) (Teile) | B | C | D | E | F | G | Endstoff I (H) | % der Theorie | Kp °C/mbar |
|---|---|---|---|---|---|---|---|---|---|---|---|---|
| 2 | – | Cl | 510 | 1500 | 360 | 11 | 675 | 229 | 1000 | 544 | 96 | 136/32 |
| 3 | – | $CF_3$ | 483 | 1300 | 276 | 10 | 470 | 174 | 1000 | 448 | 85 | 61–63/0,5 |
| 4 | 4-F | Cl | 145,5 | 500 | 92 | 5 | 160 | 58 | 400 | 149,7 | 94 | 104/1,5 |
| 5 | – | $OCH_3$ | 375 | 1000 | 276 | 10 | 500 | 184 | 800 | 340 | 82 | 68/0,1 |
| 6 | 4-F | $CH_3$ | 145,5 | 1000 | 132 | 4 | 190 | 70 | 800 | 147 | 91 | 57/0,4 |

**Patentanspruch**

Verfahren zur Herstellung von m-substituierten N-Methylanilinen der Formel

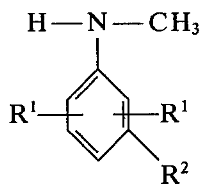

I

worin die einzelnen Reste $R^1$ und $R^2$ gleich oder verschieden sein können und jeweils einen aliphatischen Rest, eine Nitrogruppe, Alkoxygruppe, ein Halogenatom bedeuten, $R^1$ auch ein Wasserstoffatom bezeichnet, durch Alkylierung von Anilinen in Gegenwart von quaternären Ammoniumsalzen oder Phosphoniumsalzen, dadurch gekennzeichnet, daß man m-substituierte Aniline der Formel

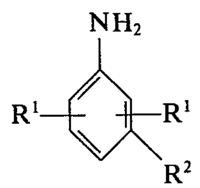

II

worin $R^1$ und $R^2$ die vorgenannte Bedeutung besitzen,

a) mit Ameisensäure in Gegenwart unter den Reaktionsbedingungen inerter, organischer Lösungsmittel bei einer Temperatur unterhalb 140° C umsetzt, anschließend
b) das Gemisch in Gegenwart eines quaternären Salzes der Formel

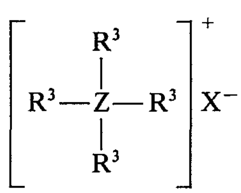

III

worin Z ein Stickstoffatom oder ein Phosphoratom bedeutet, X ein Säureanion bezeichnet und die einzelnen Reste $R^3$ gleich oder verschieden sein können und jeweils für einen aliphatischen, cycloaliphatischen, araliphatischen oder aromatischen Rest stehen, mit einem Methylierungsmittel und Alkalihydroxid bei einer Temperatur von 0 bis 50° C umsetzt und dann
c) das Gemisch bei einer Temperatur oberhalb von 50° C und einem pH oberhalb 13 umsetzt.

**Claim**

A process for the production of m-substituted N-methylanilines of the formula

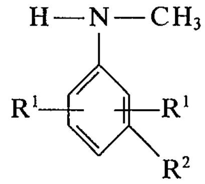

I

where the individual radicals $R^1$ and $R^2$ may be identical or different and each denotes an aliphatic radical, a nitro group, an alkoxy group or a halogen atom, $R^1$ also denoting a hydrogen atom, by alkylating anilines in the presence of quaternary ammonium salts or phosphonium salts, characterized in that m-substituted anilines of the formula

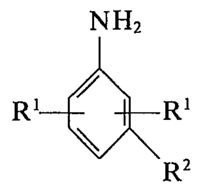

II

where $R^1$ and $R^2$ have the above meanings,

(a) are reacted with formic acid in the presence of organic solvents which are inert under the reaction conditions, at a temperature below 140° C, following which
(b) the mixture is reacted with a methylating agent and an alkali metal hydroxide at a temperature of from 0° to 50° C in the presence of a quaternary salt of the formula

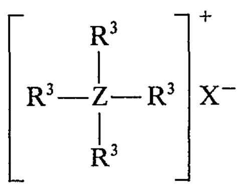

III

where Z is a nitrogen or phosphorus atom, X is an acid anion, and the individual radicals $R^3$ may be identical or different and each denotes an aliphatic, cycloaliphatic, araliphatic or aromatic radical, and then
(c) the mixture is reacted at a temperature above 50° C and a pH above 13.

**Revendication**

Procédé de préparation de N-méthylanilines substituées en méta répondant à la formule

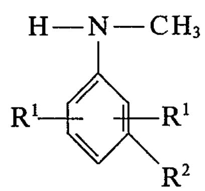

I

dans laquelle les divers symboles $R^1$ et $R^2$, ayant des significations identiques ou différentes, représentent chacun un reste aliphatique, un groupe nitro, un groupe alcoxy, un atome d'halogène, $R^1$ représentant également un atome d'hydrogène, par alkylation d'anilines en présence de sels d'ammonium ou de sels de phosphonium quaternaires, caractérisé en ce que, partant d'anilines substituées en méta répondant à la formule

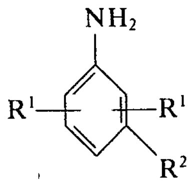

II

dans laquelle $R^1$ et $R^2$ ont les significations indiquées ci-dessus,

a) on les fait réagir avec l'acide formique en présence de solvants organiques inertes dans les conditions de la réaction à une température inférieure à 140° C, puis

b) on fait réagir le mélange en présence d'un sel quaternaire de formule

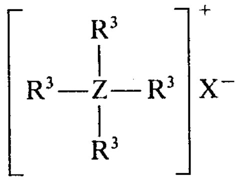

III

dans laquelle Z représente un atome d'azote ou un atome de phosphore, X représente un anion acide et les divers symboles $R^3$, ayant des significations identiques ou différentes, représentent chacun un reste aliphatique, cycloaliphatique, araliphatique ou aromatique, avec un agent méthylant et un hydroxyde alcalin, à une température de 0 à 50° C, puis

c) on fait réagir le mélange à une température supérieure à 50° C et à un pH supérieur à 13.